# EUROPEAN PATENT APPLICATION

(11) **EP 4 458 978 A1**
(43) Date of publication of application: **06.11.2024**
(21) Application number: 22914818.4
(22) Date of filing: 27.12.2022
(51) Int. Cl.: C12P 1/02, C12N 1/06, C12N 1/16, A23L 31/10

(54) **YEAST PROTEIN HAVING ANTIBACTERIAL FUNCTION AND PREPARATION METHOD THEREFOR**

(30) Priority: 28.12.2021 CN 202111631106
(71) Applicant: Angel Yeast Co., Ltd, Yichang, Hubei 443003 (CN)
(72) Inventor: QIN, Xianwu, Yichang, Hubei 443003 (CN); XU, Zhipeng, Yichang, Hubei 443003 (CN); HU, Junpeng, Yichang, Hubei 443003 (CN); DAI, Jinjun, Yichang, Hubei 443003 (CN); XIE, Zhiwen, Yichang, Hubei 443003 (CN); HUANG, Xin, Yichang, Hubei 443003 (CN)
(74) Representative: Zacco GmbH
(86) International application number: PCT/CN2022/142484
(87) International publication number: WO 2023/125566

(57) **Abstract**

Provided in the present disclosure are a yeast protein having an antibacterial function and a preparation method therefor. The method includes: S1, performing liquid fermentation on a *Saccharomyces cerevisiae* strain, and then performing separation to obtain a yeast milk; S2, acidifying the yeast milk to obtain an acidified yeast milk; and optionally performing autolysis, or exogenous enzyme catalytic hydrolysis, or mechanical crushing on the yeast milk to obtain a cell wall emulsion; S3, mixing the acidified yeast milk and the optional cell wall emulsion and performing autolysis to obtain an autolysis mixed solution; S4, performing first enzymolysis with compound protease to obtain a first enzymolysis solution; S5, performing second enzymolysis with seminase to obtain a second enzymolysis solution; and performing third enzymolysis with β-glucanase and cellulase to obtain a yeast protein emulsion; and S6, evaporating and concentrating the yeast protein emulsion to obtain the yeast protein having an antibacterial function. According to the method, a yeast protein having a broad-spectrum antibacterial function can be prepared.

## Description

### Cross-Reference to Related Application

The present disclosure claims priority to Chinese Patent Application No. 202111631106.8 filed on December 28, 2021, the disclosure of which is hereby incorporated by reference in its entirety.

### Technical Field

The present disclosure relates to the field of yeast deep processing, and specifically to a yeast protein having an antibacterial function and a preparation method therefor.

### Background

With the rapid development of animal husbandry and aquaculture in China, the need for feed is increasing, the contradiction between supply and demand of feed protein sources is becoming more and more prominent, and the shortage of protein raw materials in China has become inevitable, such that the situation of mainly relying on imports will not change. However, it has always been necessary to import a large number of imported high-protein feed raw materials from abroad, bringing enormous economic pressure to the industry on the one hand and serious challenges to biosafety on the other hand.

Since yeast hydrolysate and yeast cell are introduced into the feed raw material catalog, the yeast hydrolysate and the yeast cell wall have become more and more recognized by the industry, and the use of the yeast hydrolysate and the yeast cell wall has increased. Using yeast hydrolysate yeast protein raw materials can not only relieve the contradiction of the shortage of the feed protein resources, but also improve the nutritional quality of protein raw materials. The use effect of the yeast cell wall to enhance immunity and promote the health of animal bodies has been gradually accepted as an ideal alternative to feed antibiotics.

A current yeast hydrolysate production technology can well provide a nutritional growth promotion function in the field of farming, but has no antibacterial effect. As the era of antibiotic-free farming comes, it is of profound significance to develop a yeast protein product having an antibacterial function to realize the inhibition of bacteria such as gram-negative bacteria (Escherichia coli) and gram-positive bacteria (Staphylococcus aureus), and to realize a broad-spectrum antibacterial effect, thereby realizing the upgrading of yeast protein series products in terms of product functions. In antibiotic-free farming, a yeast protein having an antibacterial function can provide nutritions to promote growth and prevent bacterial diseases, so as to promote growth while enhancing immunity, thereby facilitating full reduction in the impact of antibiotic bans in farming.

Based on the above reasons, providing a method for preparing yeast hydrolysate having an antibacterial function is an urgent problem to be solved in the art.

### Summary

The present disclosure is mainly intended to provide a yeast protein having an antibacterial function and a preparation method therefor, so as to solve the technical problems that current yeast hydrolysate and yeast cell wall products do not have an antibacterial function and it is difficult to prevent bacterial diseases during antibiotic-free farming.

In order to implement the above objective, one aspect of the present disclosure provides a preparation method for a yeast protein having an antibacterial function, including the following steps: S1, performing liquid fermentation on a *Saccharomyces cerevisiae* strain, and then performing separation to obtain a yeast milk, wherein the *Saccharomyces cerevisiae* strain is selected from *Saccharomyces cerevisiae* FX-2 CCTCC NO: M2016418 and/or *Saccharomyces cerevisiae* d8.8 CCTCC NO: M2017148; S2, acidifying the yeast milk to obtain an acidified yeast milk; and optionally performing autolysis, or exogenous enzyme catalytic hydrolysis, or mechanical crushing on the yeast milk to obtain a cell wall emulsion; S3, mixing the acidified yeast milk and the optional cell wall emulsion and performing autolysis to obtain an autolysis mixed solution, wherein a concentration of the autolysis mixed solution is 10%-20% by mass of dry yeast; S4, performing first enzymolysis on the autolysis mixed solution with compound protease to obtain a first enzymolysis solution; S5, performing second enzymolysis on the first enzymolysis solution with seminase to obtain a second enzymolysis solution; and performing third enzymolysis on the second enzymolysis solution with β-glucanase and cellulase to obtain a yeast protein emulsion; and S6, evaporating and concentrating the yeast protein emulsion to obtain the yeast protein having an antibacterial function.

Further, in S1, a carbon source, a nitrogen source, and a phosphorus source are added by using a fed-batch method during the liquid fermentation, the carbon source is molasses, the nitrogen source is ammonia water, and the phosphorus source is an ammonium dihydrogen phosphate solution; preferably, a total sugar content in the molasses is 28.5%-31.5%, a nitrogen content in the ammonia water is 15%-17%, and a phosphorus content in the ammonium dihydrogen phosphate solution is 9%-11 %; preferably, a temperature during the liquid fermentation is 30°C-35 °C, a fermentation time is 12h-16h, and a pH value of fermentation is 4.5-6.0; and preferably, a protein content in the yeast milk is greater than or equal to 50%.

Further, S1 includes: performing the liquid fermentation on the *Saccharomyces cerevisiae* d8.8 CCTCC NO: M2017148, and then performing separation to obtain a first yeast milk; and performing the liquid fermentation on the *Saccharomyces cerevisiae* FX-2 CCTCC NO: M2016418, and then performing separation to obtain a second yeast milk; in S2, the first yeast milk is acidified to obtain the acidified yeast milk; and the autolysis, or the exogenous enzyme catalytic hydrolysis, or the mechanical crushing is performed on the second yeast milk to obtain the cell wall emulsion; and in S3, the acidified yeast milk and the cell wall emulsion are mixed and subjected to autolysis, so as to obtain the autolysis mixed solution.

Further, in S2, the first yeast milk is acidified by using citric acid; preferably, an addition amount of the citric acid is 1%-4% of the weight of the yeast milk; preferably, in S2, a protein content in the cell wall emulsion is 15%-30%; preferably, S3 includes: mixing the acidified yeast milk and the cell wall emulsion to obtain a premixed solution with a protein content greater than or equal to 35%; and adjusting a solid content of the premixed solution, and then performing heating with steam to perform autolysis, so as to obtain the autolysis mixed solution; and more preferably, in the process of performing heating with steam to perform autolysis, a temperature is controlled at 45 °C-55 °C, and a temperature-holding time is 8h-14h.

Further, the compound protease includes papain, neutral protease, alkaline protease, and acid protease; and preferably, the compound protease is protease EF108.

Further, in the first enzymolysis process, an addition amount of the compound protease is 4‰-8‰ of the weight of dry bases in the autolysis mixed solution; and preferably, an enzymolysis temperature of the first enzymolysis process is 55 °C-60 °C, a pH is 5.0-6.0, and an enzymolysis time is 6h-12h.

Further, in the second enzymolysis process, an addition amount of the seminase is 4‰-8‰ of the weight of dry bases in the first enzymolysis solution; and preferably, an enzymolysis temperature of the second enzymolysis process is 55 °C-60 °C, a pH is 5.0-6.0, and an enzymolysis time is 6h-12h.

Further, in the third enzymolysis process, an addition amount of the β-glucanase is 3‰-6‰ of the weight of dry bases in the second enzymolysis solution, and an addition amount of the cellulase is 1‰-3‰ of the weight of the dry bases in the second enzymolysis solution; preferably, an enzymolysis temperature of the third enzymolysis process is 45 °C-55 °C, a pH is 4.0-5.0, and an enzymolysis time is 8h-16h; more preferably, in the third enzymolysis process, a pH value is regulated to 4.0-5.0 by using an organic acid and/or inorganic acid; and more preferably, the organic acid is one or more of malic acid, citric acid, and lactic acid, the inorganic acid is hydrochloric acid and/or sulfuric acid, and more preferably, the pH value is regulated by using the organic acid.

Further, S6 includes: heating the yeast protein emulsion to 78 °C-82 °C to perform enzyme inactivation for 1h-2h; and evaporating and concentrating the yeast protein emulsion that has been subjected to enzyme inactivation, until a solid content reaches 35%-45%, so as to obtain the yeast protein having an antibacterial function. Preferably, after the evaporation and concentration step, the preparation method further includes: performing spray drying on a concentrated solution obtained in the evaporation and concentration step, so as to obtain the yeast protein having an antibacterial function; and more preferably, in the spray drying process, an inlet air temperature is controlled at 155 °C-175 °C, an outlet air temperature is controlled at 90 °C-115 °C, an atomization pressure is controlled at 100bar-160bar, and a material-receiving temperature is controlled at 25 °C-45°C.

Another aspect of the present disclosure further provides a yeast protein having an antibacterial function. The yeast protein is prepared by the above preparation method.

In the present disclosure, the particular *Saccharomyces cerevisiae* strain is used to obtain a yeast milk raw material and an optional yeast cell wall raw material, the acidified yeast milk and the optional yeast cell wall emulsion are mixed, then enzymolysis is performed on the mixed solution by using a particular enzymolysis technology, and finally a yeast protein product having an antibacterial function is formed through evaporation and concentration. Therefore, the antibacterial function is increased on the basis of meeting the functional indexes of yeast protein products in protein (protein being greater than or equal to 35%) and small peptide, etc., and a yeast protein product having a broad-spectrum antibacterial function is developed. The yeast protein may prevent bacterial diseases during antibiotic-free farming, so as to achieve the effect of enhancing immunity.

The *Saccharomyces cerevisiae* FX-2 is deposited with the China Center for Type Culture Collection (CCTCC) on August 1, 2016 at Wuhan University, Wuhan, China, 430072, Tel (027)-68754052, and assigned with the accession number of CCTCC NO: M2016418.

The *Saccharomyces cerevisiae* d8.8 is deposited with the China Center for Type Culture Collection (CCTCC) on March 29, 2017 at Wuhan University, Wuhan, China, 430072, Tel (027)-68754052, and assigned with the accession number of CCTCC NO: M2017148.

### Brief Description of the Drawings

The drawings described herein are used to provide a further understanding of this application, and constitute a part of the present disclosure. The exemplary embodiments and descriptions of the present disclosure are used to explain the present disclosure and do not constitute an improper limitation of the present disclosure. In the drawings:
Fig. 1 shows an experiment result of inhibition of Escherichia coli by a yeast protein prepared according to examples and comparative examples of the present disclosure.
Fig. 2 shows an experiment result of inhibition of Staphylococcus aureus by a yeast protein prepared according to examples and comparative examples of the present disclosure.

### Detailed Description of the Embodiments

It is to be noted that the embodiments in the present disclosure and the features in the embodiments may be combined with one another without conflict. The present disclosure will now be described below in detail with reference to the drawings and the embodiments.

As described in the Background, current yeast hydrolysate and yeast cell wall products have no inhibition effect on the growth of gram-negative bacteria (Escherichia coli) and gram-positive bacteria (Staphylococcus aureus), and cannot form effective bacteriostatic circles in a bacteriostatic circle test. In order to solve this problem, the present disclosure provides a preparation method for a yeast protein having an antibacterial function. In the present disclosure, functional upgrading is realized on yeast water protein products, the mixed product yeast protein inhibits bacteria such as the gram-negative bacteria (Escherichia coli) and the gram-positive bacteria (Staphylococcus aureus), and a broad-spectrum antibacterial function is given to the yeast protein.

A typical embodiment of the present disclosure provides a preparation method for a yeast protein having an antibacterial function, including the following steps: S1, performing liquid fermentation on a *Saccharomyces cerevisiae* strain, and then performing separation to obtain a yeast milk, wherein the *Saccharomyces cerevisiae* strain is selected from *Saccharomyces cerevisiae* FX-2 CCTCC NO: M2016418 and/or *Saccharomyces cerevisiae* d8.8 CCTCC NO: M2017148; S2, acidifying the yeast milk to obtain an acidified yeast milk; and optionally performing autolysis, or exogenous enzyme catalytic hydrolysis, or mechanical crushing on the yeast milk to obtain a cell wall emulsion; S3, mixing the acidified yeast milk and the optional cell wall emulsion and performing autolysis to obtain an autolysis mixed solution, wherein a concentration of the autolysis mixed solution is 10%-20% by mass of dry yeast; S4, performing first enzymolysis on the autolysis mixed solution with compound protease to obtain a first enzymolysis solution; S5, performing second enzymolysis on the first enzymolysis solution with seminase to obtain a second enzymolysis solution; and performing third enzymolysis on the second enzymolysis solution with β-glucanase and cellulase to obtain a yeast protein emulsion; and S6, evaporating and concentrating the yeast protein emulsion to obtain the yeast protein having an antibacterial function.

In the present disclosure, the particular *Saccharomyces cerevisiae* strain is used to obtain a yeast milk raw material and an optional yeast cell wall raw material (representing that the cell wall emulsion may or may not be added), the acidified yeast milk and the optional yeast cell wall emulsion are mixed, then enzymolysis is performed on the mixed solution by using a particular enzymolysis technology, and finally a yeast protein product having an antibacterial function is formed through evaporation and concentration. The yeast protein product contains proteins, nucleic acids, small peptides, glucans, mannan, and the like, has a dissolution rate that can reach 45%-65%, and improves antibacterial function on the basis of meeting the functional indexes of the yeast protein product in protein (protein being greater than or equal to 35% in mass content) and small peptide, etc., and a yeast protein product having a broad-spectrum antibacterial function is developed. The yeast protein can replace antibiotics to be applied to the field of livestock and poultry farming, and may prevent bacterial diseases, such that the impact due to antibiotic bans is reduced, and the effect of enhancing immunity is achieved.

In order to further improve the quality of the yeast milk raw material, so as to improve the performance of the final yeast protein product, in a preferred embodiment, in S1, a carbon source, a nitrogen source, and a phosphorus source are added by using a fed-batch method during the liquid fermentation, the carbon source is molasses, the nitrogen source is ammonia water, and the phosphorus source is an ammonium dihydrogen phosphate solution (aqueous solution); preferably, a total sugar content in the molasses is 28.5%-31.5% (mass content), a nitrogen content in the ammonia water is 15%-17% (mass content), and a phosphorus content in the ammonium dihydrogen phosphate solution is 9%-11% (mass content); preferably, a temperature during the liquid fermentation is 30°C-35 °C, a fermentation time is 12h-16h, and a pH value of fermentation is 4.5-6.0; and preferably, a protein content in the yeast milk is greater than or equal to 50% (mass content).

It is to be noted that, in S2, the yeast milk obtained in S1 may be divided into two portions; one portion is used for acidification to form the acidified yeast milk, and the other portion is used to prepare the cell wall emulsion; and the acidified yeast milk and the cell wall emulsion may also be prepared by respectively using different yeast milks prepared by different *Saccharomyces cerevisiae* strains. Definitely, subsequent enzymolysis may also be performed by only using the acidified yeast milk. In order to further improve the antibacterial performance of the yeast protein, in a preferred embodiment, S1 includes: performing the liquid fermentation on the *Saccharomyces cerevisiae* d8.8 CCTCC NO: M2017148, and then performing separation to obtain a first yeast milk; and performing the liquid fermentation on the *Saccharomyces cerevisiae* FX-2 CCTCC NO: M2016418, and then performing separation to obtain a second yeast milk; in S2, the first yeast milk is acidified to obtain the acidified yeast milk; and the autolysis, or the exogenous enzyme catalytic hydrolysis, or the mechanical crushing is performed on the second yeast milk to obtain the cell wall emulsion; and in S3, the acidified yeast milk and the cell wall emulsion are mixed and subjected to autolysis, so as to obtain the autolysis mixed solution.

The purpose of acidification is to form an acidification system to improve the activity of an endogenous enzyme, and in order to further improve the efficiency, preferably, in S2, the yeast milk is acidified by using citric acid; and preferably, an addition amount of the citric acid is 1%-4% of the weight of the yeast milk.

In a preferred embodiment, in S2, a protein content in the cell wall emulsion is 15%-30%; preferably, S3 includes: mixing the acidified yeast milk and the cell wall emulsion to obtain a premixed solution with a protein content greater than or equal to 35% (more preferably, by solid proportion, 5%-35% of the cell wall emulsion is added to the acidified yeast milk); and adjusting a solid content of the premixed solution, and then performing heating with steam to perform autolysis, so as to obtain the autolysis mixed solution. The acidified yeast milk and the cell wall emulsion are prepared according to the protein content greater than or equal to 35%, such that contents of various components in the final product are balanced, and at the same time, the antibacterial effect of the yeast protein product is further improved. The more stable autolysis mixed solution with more appropriate solid content is formed through adjustment, steam heating (heat shock and tank loading), and autolysis, such that the subsequent enzymolysis process is performed more stably. More preferably, in the process of performing heating to perform autolysis, a temperature is controlled at 45 °C-55 °C, and a temperature-holding time is 8h-14h. More preferably, when dry materials are regulated, the solid content in the premixed solution is adjusted to 10%-20%.

Preferably, the compound protease includes papain, neutral protease, alkaline protease, and acid protease; and preferably, the compound protease is Angel Annzyme compound enzymic preparation protease EF108. The compound protease is used to perform protease enzymolysis on the autolysis mixed solution, macromolecules contained in yeast cell are hydrolyzed by proteases into small molecules such as polypeptides, etc., such that a spatial structure is opened to expose many specific and functional groups, thereby further facilitating nutrient content and antibacterial performance of the product.

In order to cause the protease enzymolysis process to be more stable, in a preferred embodiment, in the first enzymolysis process, an addition amount of the compound protease is 4‰-8‰ of the weight of dry bases in the autolysis mixed solution; and preferably, an enzymolysis temperature of the first enzymolysis process is 55 °C-60 °C, a pH is 5.0-6.0, and an enzymolysis time is 6h-12h.

In order to cause seminase enzymolysis to be more stable, in a preferred embodiment, in the second enzymolysis process, an addition amount of the seminase is 4‰-8‰ of the weight of dry bases in the first enzymolysis solution; and preferably, an enzymolysis temperature of the second enzymolysis process is 55°C-60°C, a pH is 5.0-6.0, and an enzymolysis time is 6h-12h.

In order to cause cellulase enzymolysis to be more stable, in a preferred embodiment, in the third enzymolysis process, an addition amount of the β-glucanase is 3‰-6‰ of the weight of dry bases in the second enzymolysis solution, and an addition amount of the cellulase is 1‰-3‰ of the weight of the dry bases in the second enzymolysis solution; preferably, an enzymolysis temperature of the third enzymolysis process is 45°C-55°C, a pH is 4.0-5.0, and an enzymolysis time is 8h-16h.

Exemplarily, in the third enzymolysis process, a pH value is regulated to 4.0-5.0 by using an organic acid and/or inorganic acid; and more preferably, the organic acid is one or more of malic acid, citric acid, and lactic acid, the inorganic acid is hydrochloric acid and/or sulfuric acid, and more preferably, the pH value is regulated by using the organic acid.

In a preferred embodiment, S6 includes: heating the yeast protein emulsion to 78°C-82 °C to perform enzyme inactivation for 1h-2h; and evaporating and concentrating the yeast protein emulsion that has been subjected to enzyme inactivation, until a solid content reaches 35%-45%, so as to obtain the yeast protein having an antibacterial function. A paste product formed through evaporation and concentration also has the antibacterial effect described in the present disclosure. The specific evaporation and concentration process may be performed by using a three-effect or four-effect evaporation system.

Preferably, after the evaporation and concentration step, the preparation method further includes: performing spray drying on a concentrated solution obtained in the evaporation and concentration step, so as to obtain the yeast protein having an antibacterial function. The yeast protein is conveniently stored and transported after spray drying. The temperature of spray drying directly affects product moisture and product colors; if the temperature is controlled to be too low, the product color is light and the moisture content is high, such that it is difficult to control health indicators of the product; and if the temperature is high, the product color is dark and the moisture is low, such that it is difficult to control the quality of the product. More preferably, in the spray drying process, an inlet air temperature is controlled at 155 °C-175 °C, an outlet air temperature is controlled at 90 °C-115 °C, an atomization pressure is controlled at 100 bar-160 bar, and a material-receiving temperature (material packaging temperature) is controlled at 25 °C-45 °C.

During an actual operation, the autolysis, or the exogenous enzyme catalytic hydrolysis, or the mechanical crushing is performed on the yeast milk to obtain the cell wall emulsion. Specifically, the autolysis method is preferably used, including: performing autolysis on the yeast milk, controlling a salt concentration to be 3%-5%, a pH value to be 6.5, a temperature to be 55 °C, and after performing autolysis for 15-25 hours, performing centrifugation to obtain a yeast cell wall precipitate.

Another aspect of the present disclosure further provides a yeast protein having an antibacterial function. The yeast protein is prepared by the above preparation method. A raw material source of the yeast protein product having an antibacterial function is derived from natual yeast, such that scaled production may be realized. According to the yeast protein having an antibacterial function obtained in the present disclosure, on the basis of maintaining crude protein and small peptide nutrients, a yeast polysaccharide content is increased compared to single yeast hydrolysate, such that immunity is extra improved. Through the specific enzymolysis of the yeast protein and the yeast polysaccharide, a polysaccharide enzymolysis solution is extra used to produce an inhibition effect on bacteria while nutritional ingredient protein and functional ingredient small peptides maintain at high levels, so as to form the yeast protein having an antibacterial function. Specifically, in the present disclosure, full enzymolysis is performed on proteins in the yeast cell and cell wall by fully using the protease, many functional groups and functional components are exposed, and then enzymolysis is performed on the yeast cell wall by using polysaccharase, such that antibacterial active substances in the yeast cell and yeast cell wall are fully released to form the antibacterial effect. For the problem of antibiotics bans in current farming, in the present disclosure, developing the yeast protein having an antibacterial function can relieve a series of impacts caused by the antibiotics bans in farming, thereby promoting the growth of animals, improving a conversion rate, and increasing a farming level.

The present disclosure has passed experimental small trial, production pilot scale test, and large-scale test production, the production method is feasible.

The present disclosure is further described in detail below with reference to specific examples, and the examples cannot be construed as limiting the scope of protection claimed in the present disclosure.

### Example 1

### (1) Fermentation culture of yeast

A yeast strain was a *Saccharomyces cerevisiae* strain (*Saccharomyces cerevisiae* FX-2 CCTCC NO: M2016418, *Saccharomyces cerevisiae* d8.8, CCTCC NO: M2017148), a culture solution contained a carbon source, a nitrogen source, and a phosphorus source; the carbon source was 30% of cane molasses, the nitrogen source was ammonia water with a nitrogen content of 16%, and the phosphorus source was phosphoric acid; the *Saccharomyces cerevisiae* strain was inoculated for fermentation culture; the carbon source, the nitrogen source, and the phosphorus source were added by using a fed-batch method; a fermentation temperature was 30 °C, and a fermentation time was 17 hours; and a fermentation pH was controlled at 5.5, a yeast milk fermented by the pure culture solution was obtained, and a protein in the yeast milk was determined to be 46%.

The yeast milk prepared by the *Saccharomyces cerevisiae* d8.8 through the above process was used to prepare an acidified yeast milk: based on the mass of dry materials in the yeast milk, the yeast milk is acidified by adding 4% of citric acid, so as to obtain the acidified yeast milk.

The yeast milk prepared by the *Saccharomyces cerevisiae* FX-2 through the above process was used to prepare a cell wall emulsion: autolysis was performed on the yeast milk, a salt concentration during a yeast autolysis process was controlled to be 6%, a pH value was 6.5, a temperature was 55°C, and after 25 hours of autolysis, centrifugation was performed to obtain the yeast cell wall emulsion, with a protein content being 15%.

### (2) Mixing process of yeast milk and yeast cell wall

Based on the mass of dry materials in the yeast milk, 35% of the cell wall emulsion was added to the acidified yeast milk for mixing, so as to obtain a premixed solution with a protein content being about 35%.

### (3) Autolysis process of premixed solution

A solid content in the premixed solution was regulated to 10% with water, then heat shock and tank loading were performed, and heating and autolysis were performed. An autolysis temperature range was 45 °C, and a temperature-holding time was 14h.

### (4) Protease enzymolysis process

A complex enzyme was added to the obtained autolysis mixed solution for enzymolysis, the complex enzyme contained Angel Annzyme compound enzymic preparation protease EF108, based on the mass of dry materials in the mixed solution, an addition amount of the protease EF108 was 8‰, an enzymolysis temperature of a complex enzyme preparation AH-1 was 55 °C, a pH was 6.0, and an enzymolysis time was 12 hours.

### (5) Polysaccharase enzymolysis process

Seminase was added to the obtained protease enzymolysis mixed solution for enzymolysis, an addition amount of the seminase was 8‰, an enzymolysis temperature was 55 °C, a pH was 6.0, and an enzymolysis time was 12 hours. Then, β-glucanase and cellulase were used at the same time for further enzymolysis, an addition amount of the β-glucanase was 6‰, an addition amount of the cellulase was 3‰, an enzymolysis temperature was 45°C, a pH was 5.0, and an enzymolysis time was 16 hours.

### (6) Evaporation and concentration process

A three-effect or four-effect evaporation system was used to evaporate and concentrate a yeast hydrolysate solution that had been subjected to autolysis enzymolysis, and a solid content in a concentrated product was 35%-45%.

### (7) Spray drying

Spray drying was performed on a paste after concentration, an inlet air temperature of spray drying was controlled at 155°C-175°C, an outlet air temperature was controlled at 90°C-115°C, an atomization pressure was controlled at 100barr-160barr, and a material-receiving temperature was controlled at 25 °C-45 °C.

### Example 2

### (1) Fermentation culture of yeast

A yeast strain was a *Saccharomyces cerevisiae* strain (*Saccharomyces cerevisiae* FX-2 CCTCC NO: M2016418, d8.8 *Saccharomyces cerevisiae* d8.8, CCTCC NO: M2017148), a culture solution contained a carbon source, a nitrogen source, and a phosphorus source; the carbon source was 30% of cane molasses, the nitrogen source was ammonia water with a nitrogen content of 16%, and the phosphorus source was phosphoric acid; the *Saccharomyces cerevisiae* strain was inoculated for fermentation culture; the carbon source, the nitrogen source, and the phosphorus source were added by using a fed-batch method; a fermentation temperature was 30 °C, and a fermentation time was 16 hours; and a fermentation pH was controlled at 5.5, a yeast milk fermented by the pure culture solution was obtained, and a protein in the yeast milk was determined to be 50%.

The yeast milk prepared by the *Saccharomyces cerevisiae* d8.8 through the above process was used to prepare an acidified yeast milk: based on the mass of dry materials in the yeast milk, the yeast milk is acidified by adding 4% of citric acid, so as to obtain the acidified yeast milk.

The yeast milk prepared by the *Saccharomyces cerevisiae* FX-2 through the above process was used to prepare a cell wall emulsion: autolysis was performed on the yeast milk, a salt concentration during a yeast autolysis process was controlled to be 6%, a pH value was 6.5, a temperature was 55°C, and after 25 hours of autolysis, centrifugation was performed to obtain the yeast cell wall emulsion, with a protein content being 15%.

### (2) Mixing process of yeast milk and yeast cell wall

Based on the mass of dry materials in the yeast milk, 5% of the cell wall emulsion was added to the acidified yeast milk for mixing, so as to obtain a premixed solution with a protein content being about 36%.

### (3) Autolysis process of mixed solution

Dry materials in the mixed solution were adjusted, then heat shock and tank loading were performed, and heating and autolysis were performed. Contents of the dry materials in the mixed solution were adjusted to 20% before autolysis. An autolysis temperature range was 55°C, and a temperature-holding time was 8h.

### (4) Protease enzymolysis process

A complex enzyme was added to the obtained autolysis mixed solution for enzymolysis, the complex enzyme contained Angel Annzyme compound enzymic preparation protease EF108, based on the mass of dry materials in the mixed solution, an addition amount of the protease EF108 was 4‰, an enzymolysis temperature of a complex enzyme preparation AH-1 was 60°C, a pH was 5.0, and an enzymolysis time was 6 hours.

### (5) Polysaccharase enzymolysis process

Seminase was added to the obtained protease enzymolysis mixed solution for enzymolysis, an addition amount of the seminase was 4‰, an enzymolysis temperature was 60 °C, a pH was 5.0, and an enzymolysis time was 6 hours. Then, β-glucanase and cellulase were used at the same time for further enzymolysis, an addition amount of the β-glucanase was 3‰, an addition amount of the cellulase was 1‰, an enzymolysis temperature was 55°C, a pH was 4.0, and an enzymolysis time was 8 hours.

### (6) Evaporation and concentration process

A three-effect or four-effect evaporation system was used to evaporate and concentrate a yeast hydrolysate solution that had been subjected to autolysis enzymolysis, and a solid content in a concentrated product was 35%-45%.

### (7) Spray drying

Spray drying was performed on a paste after concentration, an inlet air temperature of spray drying was controlled at 155°C-175°C, an outlet air temperature was controlled at 90°C-115°C, an atomization pressure was controlled at 100barr-160barr, and a material-receiving temperature was controlled at 25 °C-45 °C.

### Example 3

### (1) Fermentation culture of yeast

A yeast strain was a *Saccharomyces cerevisiae* strain (*Saccharomyces cerevisiae* FX-2 CCTCC NO: M2016418, d8.8Saccharomyces cerevisiae d8.8, CCTCC NO: M2017148), a culture solution contained a carbon source, a nitrogen source, and a phosphorus source; the carbon source was 30% of cane molasses, the nitrogen source was ammonia water with a nitrogen content of 16%, and the phosphorus source was phosphoric acid; the *Saccharomyces cerevisiae* strain was inoculated for fermentation culture; the carbon source, the nitrogen source, and the phosphorus source were added by using a fed-batch method; a fermentation temperature was 30 °C, and a fermentation time was 16 hours; and a fermentation pH was controlled at 5.5, a yeast milk fermented by the pure culture solution was obtained, and a protein in the yeast milk was determined to be 50%.

The yeast milk prepared by the *Saccharomyces cerevisiae* d8.8 through the above process was used to prepare an acidified yeast milk: based on the mass of dry materials in the yeast milk, the yeast milk is acidified by adding 1% of citric acid, so as to obtain the acidified yeast milk.

The yeast milk prepared by the *Saccharomyces cerevisiae* FX-2 through the above process was used to prepare a cell wall emulsion: autolysis was performed on the yeast milk, a salt concentration during a yeast autolysis process was controlled to be 3%, a pH value was 6.5, a temperature was 55°C, and after 15 hours of autolysis, centrifugation was performed to obtain the yeast cell wall emulsion, with a protein content being about 30%.

### (2) Mixing process of yeast milk and yeast cell wall

Based on the mass of dry materials in the yeast milk, 30% of the cell wall emulsion was added to the acidified yeast milk for mixing, so as to obtain a premixed solution with a protein content being about 45%.

### (3) Autolysis process of premixed solution

A solid content in the premixed solution was regulated to 10% with water, then heat shock and tank loading were performed, and heating and autolysis were performed. An autolysis temperature range was 45 °C, and a temperature-holding time was 14h.

### (4) Protease enzymolysis process

A complex enzyme was added to the obtained autolysis mixed solution for enzymolysis, the complex enzyme contained Angel Annzyme compound enzymic preparation protease EF108, based on the mass of dry materials in the mixed solution, an addition amount of the protease EF108 was 8‰, an enzymolysis temperature of a complex enzyme preparation AH-1 was 55 °C, a pH was 6.0, and an enzymolysis time was 12 hours.

### (5) Polysaccharase enzymolysis process

Seminase was added to the obtained protease enzymolysis mixed solution for enzymolysis, an addition amount of the seminase was 8‰, an enzymolysis temperature was 55 °C, a pH was 6.0, and an enzymolysis time was 12 hours. Then, β-glucanase and cellulase were used at the same time for further enzymolysis, an addition amount of the β-glucanase was 6‰, an addition amount of the cellulase was 3‰, an enzymolysis temperature was 45°C, a pH was 5.0, and an enzymolysis time was 16 hours.

### (6) Evaporation and concentration process

A three-effect or four-effect evaporation system was used to evaporate and concentrate a yeast hydrolysate solution that had been subjected to autolysis enzymolysis, and a solid content in a concentrated product was 35%-45%.

### (7) Spray drying

Spray drying was performed on a paste after concentration, an inlet air temperature of spray drying was controlled at 155°C-175°C, an outlet air temperature was controlled at 90°C-115°C, an atomization pressure was controlled at 100barr-160barr, and a material-receiving temperature was controlled at 25 °C-45 °C.

### Example 4

### (1) Fermentation culture of yeast

A yeast strain was a *Saccharomyces cerevisiae* strain (*Saccharomyces cerevisiae* FX-2 CCTCC NO: M2016418, d8.8 *Saccharomyces cerevisiae* d8.8, CCTCC NO: M2017148), a culture solution contained a carbon source, a nitrogen source, and a phosphorus source; the carbon source was 30% of cane molasses, the nitrogen source was ammonia water with a nitrogen content of 16%, and the phosphorus source was phosphoric acid; the *Saccharomyces cerevisiae* strain was inoculated for fermentation culture; the carbon source, the nitrogen source, and the phosphorus source were added by using a fed-batch method; a fermentation temperature was 30 °C, and a fermentation time was 12 hours; and a fermentation pH was controlled at 5.5, a yeast milk fermented by the pure culture solution was obtained, and a protein in the yeast milk was determined to be 60%.

### (2) Acidification of yeast milk

Based on the mass of dry materials in the yeast milk, the yeast milk is acidified by adding 1% of citric acid, so as to obtain the acidified yeast milk. A yeast cell wall emulsion was not added.

### (3) Autolysis process

Dry materials in the acidified yeast milk were adjusted, then heat shock and tank loading were performed, and heating and autolysis were performed. Contents of the dry materials in the mixed solution were adjusted to 10% before autolysis. An autolysis temperature range was 45°C, and a temperature-holding time was 14h.

### (4) Protease enzymolysis process

A complex enzyme was added to the obtained autolysis mixed solution for enzymolysis, the complex enzyme contained Angel Annzyme compound enzymic preparation protease EF108, based on the mass of dry materials in the mixed solution, an addition amount of the protease EF108 was 8‰, an enzymolysis temperature of a complex enzyme preparation AH-1 was 55 °C, a pH was 6.0, and an enzymolysis time was 12 hours.

### (5) Polysaccharase enzymolysis process

Seminase was added to the obtained protease enzymolysis mixed solution for enzymolysis, an addition amount of the seminase was 8‰, an enzymolysis temperature was 55 °C, a pH was 6.0, and an enzymolysis time was 12 hours. Then, β-glucanase and cellulase were used at the same time for further enzymolysis, an addition amount of the β-glucanase was 6‰, an addition amount of the cellulase was 3‰, an enzymolysis temperature was 45°C, a pH was 5.0, and an enzymolysis time was 16 hours.

### (6) Evaporation and concentration process

A three-effect or four-effect evaporation system was used to evaporate and concentrate a yeast hydrolysate solution that had been subjected to autolysis enzymolysis, and a solid content in a concentrated product was 35%-45%.

### (7) Spray drying

Spray drying was performed on a paste after concentration, an inlet air temperature of spray drying was controlled at 155°C-175°C, an outlet air temperature was controlled at 90°C-115°C, an atomization pressure was controlled at 100barr-160barr, and a material-receiving temperature was controlled at 25 °C-45 °C.

### Example 5

### (1) Fermentation culture of yeast

A yeast strain was a *Saccharomyces cerevisiae* strain (*Saccharomyces cerevisiae* FX-2 CCTCC NO: M2016418, d8.8 *Saccharomyces cerevisiae* d8.8, CCTCC NO: M2017148), a culture solution contained a carbon source, a nitrogen source, and a phosphorus source; the carbon source was 30% of cane molasses, the nitrogen source was ammonia water with a nitrogen content of 16%, and the phosphorus source was phosphoric acid; the *Saccharomyces cerevisiae* strain was inoculated for fermentation culture; the carbon source, the nitrogen source, and the phosphorus source were added by using a fed-batch method; a fermentation temperature was 30 °C, and a fermentation time was 12 hours; and a fermentation pH was controlled at 5.5, a yeast milk fermented by the pure culture solution was obtained, and a protein in the yeast milk was determined to be 60%.

The yeast milk prepared by the *Saccharomyces cerevisiae* d8.8 through the above process was used to prepare an acidified yeast milk: based on the mass of dry materials in the yeast milk, the yeast milk is acidified by adding 4% of citric acid, so as to obtain the acidified yeast milk.

The yeast milk prepared by the *Saccharomyces cerevisiae* FX-2 through the above process was used to prepare a cell wall emulsion: autolysis was performed on the yeast milk, a salt concentration during a yeast autolysis process was controlled to be 3%, a pH value was 6.5, a temperature was 55°C, and after 15 hours of autolysis, centrifugation was performed to obtain the yeast cell wall emulsion, with a protein content being about 30%.

### (2) Mixing process of yeast milk and yeast cell wall

Based on the mass of dry materials in the yeast milk, 30% of the cell wall emulsion was added to the acidified yeast milk for mixing, so as to obtain a premixed solution with a protein content being about 50%.

### (3) Autolysis process of mixed solution

A solid content in the premixed solution was regulated to 20% with water, then heat shock and tank loading were performed, and heating and autolysis were performed. An autolysis temperature range was 55°C, and a temperature-holding time was 8h.

### (4) Protease enzymolysis process

A complex enzyme was added to the obtained autolysis mixed solution for enzymolysis, the complex enzyme contained Angel Annzyme compound enzymic preparation protease EF108, based on the mass of dry materials in the mixed solution, an addition amount of the protease EF108 was 4‰, an enzymolysis temperature of a complex enzyme preparation AH-1 was 60°C, a pH was 5.0, and an enzymolysis time was 6 hours.

### (5) Polysaccharase enzymolysis process

Seminase was added to the obtained protease enzymolysis mixed solution for enzymolysis, an addition amount of the seminase was 4‰, an enzymolysis temperature was 60 °C, a pH was 5.0, and an enzymolysis time was 6 hours. Then, β-glucanase and cellulase were used at the same time for further enzymolysis, an addition amount of the β-glucanase was 3‰, an addition amount of the cellulase was 1‰, an enzymolysis temperature was 55°C, a pH was 4.0, and an enzymolysis time was 8 hours.

### (6) Evaporation and concentration process

A three-effect or four-effect evaporation system was used to evaporate and concentrate a yeast hydrolysate solution that had been subjected to autolysis enzymolysis, and a solid content in a concentrated product was 35%-45%.

### (7) Spray drying

Spray drying was performed on a paste after concentration, an inlet air temperature of spray drying was controlled at 155°C-175°C, an outlet air temperature was controlled at 90 °C-115 °C, an atomization pressure was controlled at 100barr-160barr, and a material-receiving temperature was controlled at 25 °C-45 °C.

### Example 6

### (1) Fermentation culture of yeast

A yeast strain was a *Saccharomyces cerevisiae* strain (*Saccharomyces cerevisiae* FX-2 CCTCC NO: M2016418, d8.8 *Saccharomyces cerevisiae* d8.8, CCTCC NO: M2017148), a culture solution contained a carbon source, a nitrogen source, and a phosphorus source; the carbon source was 30% of cane molasses, the nitrogen source was ammonia water with a nitrogen content of 16%, and the phosphorus source was phosphoric acid; the *Saccharomyces cerevisiae* strain was inoculated for fermentation culture; the carbon source, the nitrogen source, and the phosphorus source were added by using a fed-batch method; a fermentation temperature was 30 °C, and a fermentation time was 14 hours; and a fermentation pH was controlled at 5.5, a yeast milk fermented by the pure culture solution was obtained, and a protein in the yeast milk was determined to be 55%.

The yeast milk prepared by the *Saccharomyces cerevisiae* d8.8 through the above process was used to prepare an acidified yeast milk: based on the mass of dry materials in the yeast milk, the yeast milk is acidified by adding 2% of citric acid, so as to obtain the acidified yeast milk.

The yeast milk prepared by the *Saccharomyces cerevisiae* FX-2 through the above process was used to prepare a cell wall emulsion: autolysis was performed on the yeast milk, a salt concentration during a yeast autolysis process was controlled to be 4%, a pH value was 6.5, a temperature was 55°C, and after 20 hours of autolysis, centrifugation was performed to obtain the yeast cell wall emulsion, with a protein content being about 20%.

### (2) Mixing process of yeast milk and yeast cell wall

Based on the mass of dry materials in the yeast milk, 20% of the cell wall emulsion was added to the acidified yeast milk for mixing, so as to obtain a premixed solution with a protein content being about 48%.

### (3) Autolysis process of premixed solution

A solid content in the premixed solution was regulated to 15% with water, then heat shock and tank loading were performed, and heating and autolysis were performed. An autolysis temperature range was 50°C, and a temperature-holding time was 10h.

### (4) Protease enzymolysis process

A complex enzyme was added to the obtained autolysis mixed solution for enzymolysis, the complex enzyme contained Angel Annzyme compound enzymic preparation protease EF108, based on the mass of dry materials in the mixed solution, an addition amount of the protease EF108 was 6‰, an enzymolysis temperature of a complex enzyme preparation AH-1 was 57°C, a pH was 5.5, and an enzymolysis time was 10 hours.

### (5) Polysaccharase enzymolysis process

Seminase was added to the obtained protease enzymolysis mixed solution for enzymolysis, an addition amount of the seminase was 6‰, an enzymolysis temperature was 57 °C, a pH was 5.5, and an enzymolysis time was 10 hours. Then, β-glucanase and cellulase were used at the same time for further enzymolysis, an addition amount of the β-glucanase was 5‰, an addition amount of the cellulase was 2‰, an enzymolysis temperature was 50 °C, a pH was 4.5, and an enzymolysis time was 12 hours.

### (6) Evaporation and concentration process

A three-effect or four-effect evaporation system was used to evaporate and concentrate a yeast hydrolysate solution that had been subjected to autolysis enzymolysis, and a solid content in a concentrated product was 35%-45%.

### (7) Spray drying

Spray drying was performed on a paste after concentration, an inlet air temperature of spray drying was controlled at 155°C-175°C, an outlet air temperature was controlled at 90°C-115°C, an atomization pressure was controlled at 100barr-160barr, and a material-receiving temperature was controlled at 25 °C-45 °C.

### Example 7

### (1) Fermentation culture of yeast

A yeast strain was a *Saccharomyces cerevisiae* strain (*Saccharomyces cerevisiae* FX-2 CCTCC NO: M2016418, d8.8 *Saccharomyces cerevisiae* d8.8, CCTCC NO: M2017148), a culture solution contained a carbon source, a nitrogen source, and a phosphorus source; the carbon source was 30% of cane molasses, the nitrogen source was ammonia water with a nitrogen content of 16%, and the phosphorus source was phosphoric acid; the *Saccharomyces cerevisiae* strain was inoculated for fermentation culture; the carbon source, the nitrogen source, and the phosphorus source were added by using a fed-batch method; a fermentation temperature was 30 °C, and a fermentation time was 14 hours; and a fermentation pH was controlled at 5.5, a yeast milk fermented by the pure culture solution was obtained, and a protein in the yeast milk was determined to be 55%.

The yeast milk prepared by the *Saccharomyces cerevisiae* d8.8 through the above process was used to prepare an acidified yeast milk: based on the mass of dry materials in the yeast milk, the yeast milk is acidified by adding 3% of citric acid, so as to obtain the acidified yeast milk.

The yeast milk prepared by the *Saccharomyces cerevisiae* FX-2 through the above process was used to prepare a cell wall emulsion: autolysis was performed on the yeast milk, a salt concentration during a yeast autolysis process was controlled to be 4%, a pH value was 6.5, a temperature was 55°C, and after 20 hours of autolysis, centrifugation was performed to obtain the yeast cell wall emulsion, with a protein content being about 20%.

### (2) Mixing process of yeast milk and yeast cell wall

Based on the mass of dry materials in the yeast milk, 10% of the cell wall emulsion was added to the acidified yeast milk for mixing, so as to obtain a premixed solution with a protein content being about 50%.

### (3) Autolysis process of mixed solution

Dry materials in the mixed solution were adjusted, then heat shock and tank loading were performed, and heating and autolysis were performed. Contents of the dry materials in the mixed solution were adjusted to 15% before autolysis. An autolysis temperature range was 50°C, and a temperature-holding time was 10h.

### (4) Protease enzymolysis process

A complex enzyme was added to the obtained autolysis mixed solution for enzymolysis, the complex enzyme contained Angel Annzyme compound enzymic preparation protease EF108, based on the mass of dry materials in the mixed solution, an addition amount of the protease EF108 was 6‰, an enzymolysis temperature of a complex enzyme preparation AH-1 was 57°C, a pH was 5.5, and an enzymolysis time was 8 hours.

### (5) Polysaccharase enzymolysis process

Seminase was added to the obtained protease enzymolysis mixed solution for enzymolysis, an addition amount of the seminase was 6‰, an enzymolysis temperature was 57 °C, a pH was 5.5, and an enzymolysis time was 8 hours. Then, β-glucanase and cellulase were used at the same time for further enzymolysis, an addition amount of the β-glucanase was 4‰, an addition amount of the cellulase was 2‰, an enzymolysis temperature was 50°C, a pH was 4.5, and an enzymolysis time was 12 hours.

### (6) Evaporation and concentration process

A three-effect or four-effect evaporation system was used to evaporate and concentrate a yeast hydrolysate solution that had been subjected to autolysis enzymolysis, and a solid content in a concentrated product was 35%-45%.

### (7) Spray drying

Spray drying was performed on a paste after concentration, an inlet air temperature of spray drying was controlled at 155°C-175°C, an outlet air temperature was controlled at 90°C-115°C, an atomization pressure was controlled at 100barr-160barr, and a material-receiving temperature was controlled at 25 °C-45 °C.

### Comparative example 1

### (1) Fermentation culture of yeast

A yeast strain was a *Saccharomyces cerevisiae* strain (*Saccharomyces cerevisiae* FX-2 CCTCC NO: M2016418, d8.8 *Saccharomyces cerevisiae* d8.8, CCTCC NO: M2017148), a culture solution contained a carbon source, a nitrogen source, and a phosphorus source; the carbon source was 30% of cane molasses, the nitrogen source was ammonia water with a nitrogen content of 16%, and the phosphorus source was phosphoric acid; the *Saccharomyces cerevisiae* strain was inoculated for fermentation culture; the carbon source, the nitrogen source, and the phosphorus source were added by using a fed-batch method; a fermentation temperature was 30 °C, and a fermentation time was 12 hours; and a fermentation pH was controlled at 5.5, a yeast milk fermented by the pure culture solution was obtained, and a protein in the yeast milk was determined to be 60%

### (2) Mixing process of yeast milk and yeast cell wall

Acidification of the yeast milk was not performed, and a cell wall emulsion was not added.

### (3) Autolysis process of mixed solution

Dry materials in the mixed solution were adjusted, then heat shock and tank loading were performed, and heating and autolysis were performed. Contents of the dry materials in the mixed solution were adjusted to 21% before autolysis. An autolysis temperature range was 60°C, and a temperature-holding time was 15h.

### (4) Protease enzymolysis process

A complex enzyme was added to the obtained autolysis mixed solution for enzymolysis, the complex enzyme contained Angel Annzyme compound enzymic preparation protease EF108, based on the mass of dry materials in the mixed solution, an addition amount of the protease EF108 was 9‰, an enzymolysis temperature of a complex enzyme preparation AH-1 was 65°C, a pH was 6.5, and an enzymolysis time was 13 hours.

### (5) Polysaccharase enzymolysis process

Seminase was added to the obtained protease enzymolysis mixed solution for enzymolysis, an addition amount of the seminase was 9‰, an enzymolysis temperature was 65°C, a pH was 6.5, and an enzymolysis time was 13 hours. Then, β-glucanase and cellulase were used at the same time for further enzymolysis, an addition amount of the β-glucanase was 7‰, an addition amount of the cellulase was 4‰, an enzymolysis temperature was 60 °C, a pH was 5.5, and an enzymolysis time was 17 hours.

### (6) Evaporation and concentration process

A three-effect or four-effect evaporation system was used to evaporate and concentrate a yeast hydrolysate solution that had been subjected to autolysis enzymolysis, and a solid content in a concentrated product was 35%-45%.

### (7) Spray drying

Spray drying was performed on a paste after concentration, an inlet air temperature of spray drying was controlled at 155°C-175°C, an outlet air temperature was controlled at 90°C-115°C, an atomization pressure was controlled at 100barr-160barr, and a material-receiving temperature was controlled at 25 °C-45 °C.

A supplementary note was provided, in the above embodiment process, for the selection of endpoint values, due to error in devices and manual operations, there were certain fluctuation ranges with temperature being about ± 2 °C, pH being ± 0.2, and a mass percentage was discounted by about 2%.

A method for detecting an antibacterial function was as follows.

### 1. Preparation of culture medium

MH broth and an MH agar medium were autoclaved for 20 min at 121 °C, and an MH agar plate was prepared for later use.

### 2. Strain culture

Well preserved Escherichia coli and Staphylococcus aureus were inoculated into the MH broth, incubation was performed for 12h-14h at 37°C by using a constant temperature shaking table. The resuscitated Escherichia coli and Staphylococcus aureus were inoculated into the MH broth again, and cultured for about 16h-20h. Through detection of a spectrophotometer, an OD value was between 0.4 and 0.7. A viable count was about 10¹⁰CFU/mL.

### 3. Bacteriostatic circle test

### 3.1 Preparation of bacteria-containing plate

Normal saline (autoclaved for 20 min at 121 °C) was used to dilute a pure culture bacterial solution in S2.2 to 1000x, so as to prepare bacterial suspension.

Sterilized MH agar was poured to a plate, and after the MH plate cured, 200µL of the bacterial suspension was added dropwise to each plate.

A sterilized glass rod was used to rapidly uniformly spread the bacterial solution in the entire plate, so as to prepare the bacteria-containing plate.

3.2 A liquid sample was evaporated and concentrated to a concentration of 40% of dry materials, and then added to bacteria-containing plate oxford cups (each was added with about 250µL), making a liquid surface flush with the oxford cup, and a proper amount of about 1g-3g of a solid sample was selected by directly using a medicine spoon and was placed in the bacteria-containing plate.

3.3 The plate in 3.2 was placed in a constant temperature incubator for culture for about 14h at 37 °C.

A method for testing other indicators was as follows.

Protein content: tested by using GB/T 6432-2018.

Dissolution rate: performed according to Q/AQJM2260-2014 Feed Raw Materials, *Saccharomyces Cerevisiae* Cell Wall 5.5.

Indicator analysis results were shown in Table 1.

**Table 1**

| | Example 1 | Example 2 | Example 3 | Example 4 | Example 5 | Example 6 | Example 7 | Comparati ve example 1 |
|---|---|---|---|---|---|---|---|---|
| Protein content (%) | 35 | 44 | 51 | 48 | 52 | 50 | 60 | 60 |
| Dissolution rate (%) | 45 | 60 | 57 | 48 | 52 | 48 | 65 | 70 |
| Antibacterial effect | Effective | Effective | Effective | Effective | Effective | Effective | Effective | Ineffective |

### Bacteriostatic circle diagram:

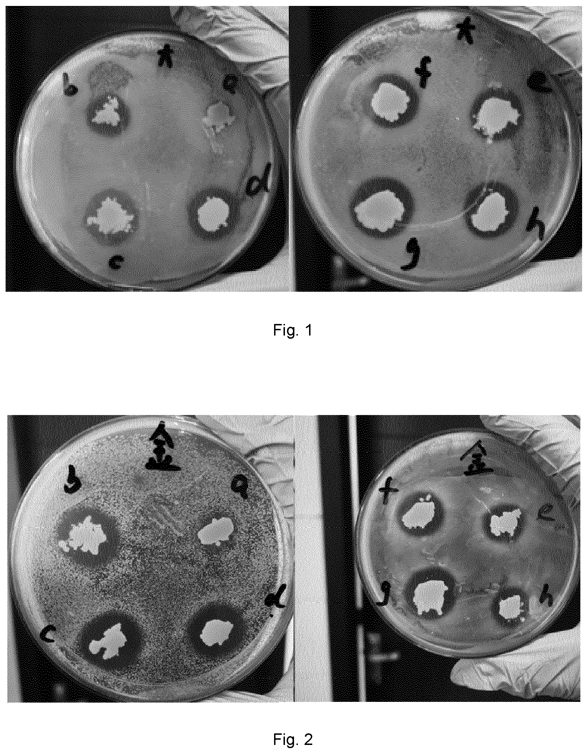

Fig. 1 showed an experiment result of inhibition of Escherichia coli by a yeast protein in the examples and comparative examples, and Fig. 2 showed an experiment result of inhibition of Staphylococcus aureus by a yeast protein in the examples and comparative examples, where a corresponded to Comparative example 1, and b-h were respectively corresponded to Examples 1-7. Results showed that for the Escherichia coli and Staphylococcus aureus, b-h all had significant bacteriostatic circles, while a had indistinct bacteriostatic circles for the Escherichia coli and Salmonella. In particular, compared with e (Example 4), the bacteriostatic circles of b, c, d, f, g, and h were more complete and wider in width, indicating that the yeast protein obtained by performing subsequent enzymolysis after the acidified yeast milk and the yeast cell wall emulsion were mixed had a better antibacterial effect.

The above are only the preferred embodiments of this application and are not intended to limit this application. For those skilled in the art, this application may have various modifications and variations. Any modifications, equivalent replacements, improvements and the like made within the spirit and principle of this application shall fall within the scope of protection of this application.

## Claims

1. A preparation method for a yeast protein having an antibacterial function, comprising the following steps:
S1, performing liquid fermentation on a *Saccharomyces cerevisiae* strain, and then performing separation to obtain a yeast milk, wherein the *Saccharomyces cerevisiae* strain is selected from *Saccharomyces cerevisiae* FX-2 CCTCC NO: M2016418 and/or *Saccharomyces cerevisiae* d8.8 CCTCC NO: M2017148;
S2, acidifying the yeast milk to obtain an acidified yeast milk; and optionally performing autolysis, or exogenous enzyme catalytic hydrolysis, or mechanical crushing on the yeast milk to obtain a cell wall emulsion;
S3, mixing the acidified yeast milk and the optional cell wall emulsion and performing autolysis to obtain an autolysis mixed solution, wherein a concentration of the autolysis mixed solution is 10%-20% by mass of dry yeast;
S4, performing first enzymolysis on the autolysis mixed solution with compound protease to obtain a first enzymolysis solution;
S5, performing second enzymolysis on the first enzymolysis solution with seminase to obtain a second enzymolysis solution; and performing third enzymolysis on the second enzymolysis solution with β-glucanase and cellulase to obtain a yeast protein emulsion; and
S6, evaporating and concentrating the yeast protein emulsion to obtain the yeast protein having an antibacterial function.

2. The preparation method according to claim 1, wherein in S1, a carbon source, a nitrogen source, and a phosphorus source are added by using a fed-batch method during the liquid fermentation, the carbon source is molasses, the nitrogen source is ammonia water, and the phosphorus source is an ammonium dihydrogen phosphate solution;
preferably, a total sugar content in the molasses is 28.5%-31.5%, a nitrogen content in the ammonia water is 15%-17%, and a phosphorus content in the ammonium dihydrogen phosphate solution is 9%-11%;
preferably, a temperature during the liquid fermentation is 30 °C-35 °C, a fermentation time is 12 h-16 h, and a pH value of fermentation is 4.5-6.0; and
preferably, a protein content in the yeast milk is greater than or equal to 50%.

3. The preparation method according to claim 1 or 2, wherein S1 comprises: performing the liquid fermentation on the *Saccharomyces cerevisiae* d8.8 CCTCC NO: M2017148, and then performing separation to obtain a first yeast milk; and performing the liquid fermentation on the *Saccharomyces cerevisiae* FX-2 CCTCC NO: M2016418, and then performing separation to obtain a second yeast milk; in S2, the first yeast milk is acidified to obtain the acidified yeast milk; and the autolysis, or the exogenous enzyme catalytic hydrolysis, or the mechanical crushing is performed on the second yeast milk to obtain the cell wall emulsion; and in S3, the acidified yeast milk and the cell wall emulsion are mixed and subjected to autolysis, so as to obtain the autolysis mixed solution.

4. The preparation method according to any one of claims 1 to 3, wherein in S2, the first yeast milk is acidified by using citric acid; preferably, an addition amount of the citric acid is 1%-4% of the weight of the yeast milk; preferably, in S2, a protein content in the cell wall emulsion is 15%-30%;
preferably, S3 comprises: mixing the acidified yeast milk and the cell wall emulsion to obtain a premixed solution with a protein content greater than or equal to 35%; and adjusting a solid content of the premixed solution, and then performing heating with steam to perform autolysis, so as to obtain the autolysis mixed solution; and
more preferably, in the process of performing heating with steam to perform autolysis, a temperature is controlled at 45 °C-55 °C, and a temperature-holding time is 8 h-14 h.

5. The preparation method according to any of claims 1 to 4, wherein the compound protease comprises papain, neutral protease, alkaline protease, and acid protease; and preferably, the compound protease is protease EF108.

6. The preparation method according to claim 6, wherein in the first enzymolysis process, an addition amount of the compound protease is 4‰-8‰ of the weight of dry bases in the autolysis mixed solution; and preferably, an enzymolysis temperature of the first enzymolysis process is 55 °C-60 °C, a pH is 5.0-6.0, and an enzymolysis time is 6 h-12 h.

7. The preparation method according to any one of claims 1 to 6, wherein in the second enzymolysis process, an addition amount of the seminase is 4‰-8‰ of the weight of dry bases in the first enzymolysis solution; and preferably, an enzymolysis temperature of the second enzymolysis process is 55 °C-60 °C, a pH is 5.0-6.0, and an enzymolysis time is 6 h-12 h.

8. The preparation method according to claim 7, wherein in the third enzymolysis process, an addition amount of the β-glucanase is 3‰-6‰ of the weight of dry bases in the second enzymolysis solution, and an addition amount of the cellulase is 1‰-3‰ of the weight of the dry bases in the second enzymolysis solution; preferably, an enzymolysis temperature of the third enzymolysis process is 45 °C-55 °C, a pH is 4.0-5.0, and an enzymolysis time is 8 h-16 h;
more preferably, in the third enzymolysis process, a pH value is regulated to 4.0-5.0 by using an organic acid and/or inorganic acid; and
more preferably, the organic acid is one or more of malic acid, citric acid, and lactic acid, the inorganic acid is hydrochloric acid and/or sulfuric acid, and more preferably, the pH value is regulated by using the organic acid.

9. The preparation method according to any one of claims 1 to 8, wherein, S6 comprises:
heating the yeast protein emulsion to 78 °C-82 °C to perform enzyme inactivation for 1 h-2 h; and
evaporating and concentrating the yeast protein emulsion that has been subjected to enzyme inactivation, until a solid content reaches 35%-45%, so as to obtain the yeast protein having an antibacterial function;
preferably, after the evaporation and concentration step, the preparation method further comprises: performing spray drying on a concentrated solution obtained in the evaporation and concentration step, so as to obtain the yeast protein having an antibacterial function; and
more preferably, in the spray drying process, an inlet air temperature is controlled at 155 °C-175°C, an outlet air temperature is controlled at 90 °C-115 °C, an atomization pressure is controlled at 100 bar-160 bar, and a material-receiving temperature is controlled at 25 °C-45 °C.

10. A yeast protein having an antibacterial function, prepared by the preparation method according to any one of claims 1 to 9.
